# EUROPÄISCHE PATENTSCHRIFT

(11) **EP 0 454 178 B1**
(45) Veröffentlichungstag und Bekanntmachung des Hinweises auf die Patenterteilung: **18.05.1994**
(21) Anmeldenummer: 91108349.1
(22) Anmeldetag: 03.02.1984
(51) Int. Cl.: A61M 1/00, A61M 1/02, A61M 5/145

(54) **Autotransfusionsgerät**
Autotransfusion apparatus
Appareil d'autotransfusion

(30) Priorität: 10.02.1983 DE 3304486
(43) Veröffentlichungstag der Anmeldung: 30.10.1991
(62) Teilanmeldung aus: 89114626.8
(73) Patentinhaber: Rühland, Dieter, Dr. med., D-78224 Singen (DE)
(72) Erfinder: Rühland, Dieter, Dr. med., D-78224 Singen (DE)
(74) Vertreter: Schumacher, Horst, Dr. Dipl.-Phys.

(56) Entgegenhaltungen:
- FR-A- 1 124 356
- FR-A- 2 346 238
- US-A- 3 896 733

## Beschreibung

Die Erfindung betrifft eine Vorrichtung gemäß dem Oberbegriff des Anspruchs 1.

Die vorliegende Anmeldung ist aus der Anmeldung 89 114 626.8 (EP-A-0 345 831); diese wiederum ist eine Ausscheidungsanmeldung der Stammanmeldung 84101 108.3 (EP-A-0 116 352).

Bei Operationen, z.B. aus dem Bereich der Herzchirurgie, Gefäßchirurgie, Unfallchirurgie oder Orthopädie kommt es häufig zu großen schnellen Blutverlusten, die heute zumeist noch durch Fremdblut ausgeglichen werden. Fremdblut ist jedoch geeignet, Krankheiten zu übertragen (z.B. Hepatits) Weiterhin fehlen dem oft mehrere Wochen alten Fremdblut die Gerinnungselemente (Gerinnungsfaktoren und Blutplättchen), die durch Lagerung zerstört werden. Es ist deshalb dringend erforderlich, Geräte zur Verfügung zu haben, die geeignet sind, daß sich in den Körperhöhlen während einer Operation ansiedelnde Blut zurückzugewinnen und dem Patienten wieder zuzuführen, um den Fremdbluteinsatz zu verringern und die Blutgerinnungselemente weitgehend zu erhalten.

Es stehen zur Rückgewinnung von Eigenblut (Autotransfusion) für große Herzoperationen komplizierte, teure Herz-Lungen-Maschinen heute zur Verfügung, die das Blut des Patienten über einen Pumpmechanismus absaugen und über lange Schläuche wieder zuführen. Andere Geräte sammeln die Blutflüssigkeit, isolieren und waschen die roten Blutzellen und führen diese gewaschenen Blutzellen dem Körper wieder zu. Bei beiden Geräten werden die empfindlichen Blutzellen durch lange Saugwege geschädigt (Oberflächenkontakt) und bei dem zweiten Gerät werden durch den Waschvorgang alle Gerinnungselemente und das Blutplasma entfernt. Diese Geräte sind zudem sehr teuer und daher nur in wenigen Zentren verfügbar.

Es sind daher weitere Geräte für die intraoperative Autotransfusion entwickelt worden. So ist aus der US-A- 4 047 526 ein Autotransfusionsgerät der eingangs genannten Art bekannt, bei dem das Blut zunächst in einem harten Behälter durch Unterdruck gesammelt und anschließend in einen mit dem harten Sammelbehälter bodenseitig lösbar verbundenen Faltbalg, mittels dessen ein Vakuum erzeugbar ist, durch welchen das Vakuum in dem darüberliegenden harten Behälter überwunden werden kann, überführt. Der mit Blut gefüllte Faltbalg wird dann von dem harten (formsteifen) Behälter gelöst und anschließend zur aktiven Rücktransfusion des Blutes verwendet.

In der US-A- 4 033 345 wird eine andere Ausführungsform eines Autotransfusionsgerätes mit einem formsteifen Zweikammersystem beschrieben, das einen innenliegenden verformbaren Beutel enthält, der mit der oberen Kammer, die zunächst das Blut sammelt, durch ein Rückschlagventil verbunden ist. Das Blut kann durch eine weitere Öffnung mit Rückschlagventil und Filtereinheiten durch Eindringen eines Druckmediums in den Raum zwischen der harten, zweiten Kammer und der äußeren Beuteloberfläche aktiv in den Patienten zurücktransfundiert werden. Die Überleitung des Blutes aus dem formsteifen Sammelbehälter in den flexiblen Beutel erfolgt durch wechselseitiges Anlegen von Über- und Unterdruck.

Bei einem aus der nachveröffentlichten DE-A- 32 18 561 bekannten, weiteren Autotransfusionsgerät handelt es sich um eine der zweiten Kammer der vorerwähnten US-A- 4 033 345 entsprechende Vorrichtung, mit der das Blut jedoch ohne Rückschlagventile durch eine Öffnung in der Kammerunterseite ausgesaugt werden muß und bei der weder ein Sieb noch ein ähnliches Blutfilter einsetzbar ist.

Schließlich ist aus der US-A- 4 014 329 ein weiteres Zweikammerautotransfusionsgerät bekannt, bei dem die erste Kammer nach dem gleichen Prinzip arbeitet, wie es in der US-A- 4 033 345 für die zweite Kammer beschrieben wurde, wobei hier aber das Blut durch seine Schwerkraft an der Kammerunterseite in die zweite Kammer mit Filter abfließt.

Mit diesen bekannten Autotransfusionsgeräten ist es zwar möglich, intraoperativ anfallendes Blut zu sammeln und in den Patienten zu retransfundieren, doch weisen sie eine Reihe entscheidender Nachteile auf: Bei den Mehrkammersystemen kommt das Blut mit einer großen Geräteoberfläche in Kontakt, was eine nachteilige Gerinnungsaktivierung und Bluttraumatisierung bewirkt. Ebenso erfolgt eine für die empfindlichen Blutzellen nachteilige, weitere Traumatisierung bei der Überführung von der einen in die andere Kammer, insbesondere wenn zwischen den Kammern Rückschlagventile eingesetzt sind. Soweit das Blut von unten in die Unterdruckkammer eingesaugt wird, wird das im Gerät bereits angesammelte Blut durch nachfolgend angesaugtes Blut in turbulente Bewegung versetzt, wobei mitangesaugte Luft und grobe Bestandteile, wie Blut, Koagel, Fettzellen und Knochensplitter, eine erhebliche Schaumbildung sowie Traumatisierung der Blutzellen bewirken. Beim Fehlen von Grobfiltern zum Zurückhalten von im Blut mitgeführten Stoffen besteht schließlich bei den herkömmlich an den Transfusionsbestecken vorgesehenen Feinfiltern eine latente Verstopfungsgefahr. Weiterhin erfordern die deckel- sowie bodenseitig mit Ein- bzw. Auslauföffnungen versehenen Blutsammelkammern eine im Hinblick auf das erforderliche Blutansaugvolumen beachtliche Bauhöhe, welche regelmäßig höher als das sehr kleine sterile Operations-Gebiet am Patienten ist; daher ergeben sich bei den bekannten Autotransfusionsgeräten regelmäßig Sterilitätsprobleme im sterilen Operations-Gebiet, die nur dadurch ausgleichbar sind, daß das Autotransfusionsgerät außerhalb des Sterilbereiches aufgestellt und ein längerer Saugweg in Kauf genommen wird. Hierdurch ist ein höherer Saugdruck erforderlich und ein vermehrter Fremdkörperkontakt gegeben - beides bedeutet ein zusätzliches Trauma für das Blut. Bei Zweikammersystemen erfordert die Überleitung des Blutes nach der Aspiration in die zweite Kammer Zeit, die weder dem Anästhesisten, noch dem Chirurgen bei einem Massenanfall von Blut zur Verfügung steht; der Zeitfaktor ist besonders aber auch für den Patienten nachteilig, da er das Blut in diesem Falle besonders schnell wieder benötigt.

Aus der FR-A-23 46 238 ist eine Blutpumpe bekannt, deren Aufgabe darin besteht, eine Blut-/Luftoberfläche bei der Blutaufnahme zu vermeiden. Zu diesem Zweck wird der Über- und Unterdruck zur Pumpenbetätigung ausschließlich auf eine der Blutsammelkammer bezüglich einer Membran gegenüberliegende Luftkammer ausgeübt, wobei sich die Einlauf- und Auslauföffnung für das Blut im Bodenteil des Pumpenbehälters befinden, während sich die Luftkammer ständig im Deckelteil befindet. Selbst wenn zusammen mit dem Blut versehentlich einmal etwas Luft angesaugt wird, wird diese im Normalfall nicht weiter transportiert und bildet auch keine, das nachströmende Blut schädigende, Blut-/Luftoberfläche, weil sich die Luft zwingendermaßen im höchsten Bereich des Bodenteiles sammelt und das weiter ein- und ausströmende Blut unterhalb dieser Oberfläche angesaugt und ausgestoßen wird, mit der Luft also nicht in Kontakt kommt. Zwischen der Zulaufleitung und der Ablaufleitung der unteren Kammer ist also eine permanente Flüssigkeitsverbindung gewährleistet. Bei eine derartigen Blutpumpe ist darauf zu achten, daß Strömungswiderstände, wie sie zum Beispiel ein Sieb in der Auslaufleitung sowie davor etwa sich ansammelnde Partikel darstellen würden, vermieden werden. Als Autotransfusionsgerät ist eine derartige Blutpumpe keinesfalls geeignet, weil mit dem Blut angesaugte Luft sowie Fette oder andere Partikel beim Reinfundieren in den Patienten nicht automatisch zurückgehalten werden können. Ein derartiges Gerät (mit Ausnahme des die Auslaßöffnung abdeckenden Siebs) liegt dem Oberbegriff des Anspruchs 1 zugrunde.

Davon ausgehend liegt der Erfindung die **Aufgabe** zugrunde, eine Vorrichtung der eingangs genannten Art zu schaffen, mit der es möglich ist, das Blut auf kurzem Wege ohne Schädigung zu sammeln und schnell bei sicherer Funktionstüchtigkeit und ohne Schädigungsmöglichkeit für den Patienten in diesen zurückzutransfundieren; insbesondere soll eine geringe Bauhöhe erreicht werden, die eine Anwendung im Sterilbereich des Patienten gestattet.

Als technische **Lösung** wird dafür die Vorrichtung mit den Merkmalen des Anspruchs 1 vorgeschlagen.

Die Erfindung beruht demnach auf dem Grundgedanken, im Deckelteil einer einzigen Blutsammelkammer sowohl die Bluteinlaßöffnung, als auch die Blutauslaßöffnung mit vorgeschaltetem Grobfilter anzuordnen, so daß das angesaugte Blut von oben in diese Kammer einströmt und ohne Schaumbildung an der Kammerwandung entlang nach unten fließt; bei der Retransfusion wird das Gerät auf den Kopf gestellt und das Blut fließt gefiltert durch Schwerkraft und/oder erhöhten Gasdruck in dem durch eine Membran von der Blutsammelkammer abgetrennten Bodenraum zurück zum Patienten; die vorangehende Blutansaugung wird dabei durch einen Unterdruck bewirkt, der entweder - bei gasdicht verschlossenem Bodenraum - an der Auslauföffnung, oder - bei gasdicht verschlossener Auslauföffnung - im Bodenraum angelegt wird; die Membran ist demnach nur an ihrem einzigen Rand festgelegt, wobei das Größenverhältnis zwischen Boden- und Deckelteil unerheblich ist und lediglich gewährleistet sein muß, daß sich die Membran alternierend sowohl an der Bodenteil- als auch an der Deckelteilwandung möglichst weitgehend anlegen kann, so daß abwechselnd der Boden und der Deckelraum ein minimales Restvolumen besitzen.

Die Einlauf- und Auslaßöffnungen müssen bei Unterdruckanwendung im Deckelraum hinreichend weit beabstandet sein, um einen Blutübertritt in die angelegten Unterdruckquelle zu vermeiden.

Das erfindungsgemäße Autotransfusionsgerät weist eine Reihe wichtiger Vorteile auf: Das Blut wird in einer einzigen Kammer ohne verengende Querschnitte , ohne Schaumbildung unter kontrolliert erzeugbarem Druck gesammelt und vorgefiltert aus derselben Kammer unter Anwendung eines frei vorgebbaren Druckes mit der gewünschten Schnelligkeit direkt in den Patienten infundiert; hierfür ist nur eine sehr geringe Bauhöhe des unterdruckfesten Behälters erforderlich, so daß dieser jederzeit im Sterilbereich der Operation handhabbar ist. Zudem werden Dichtigkeitsprobleme im Bereich der drei Behälteröffnungen und im Zusammenhang mit der Membran (Ballon) dadurch vermieden, daß diese Öffnungen allesamt in der im wesentlichen starren Wandung des Deckel bzw. Bodenteiles, und daher unabhängig vom Dichtungsrand der Membran, angeordnet sind; es braucht also lediglich der Ballon mit seinem Mündungsrand gegenüber dem Rand der Öffnung zum Herstellen einer Gasströmungsverbindung gasdicht festgelegt werden, während Durchlaßöffnungen mit Schlauchanschlüssen innerhalb der Membranfläche entfallen; hierdurch können die Membraneigenschaften und die Membranabdichtung besonders funktionsgerecht und unabhängig von der Ein- und Auslaßöffnung frei ausgewählt werden.

Es versteht sich, daß das erfindungsgemäße Autotransfusionsgerät auch derart benutzt werden kann, daß einem Patienten Blut entnommen und dieses unmittelbar anschließend einem anderen Patienten zugeführt wird.

Die Membran kann am Rand der Öffnung im Bodenteil verschweißt werden. Gemäß einer anderen Weiterbildung der Erfindung kann das Sieb, anstelle im Deckel eingeschweißt oder eingeklebt zu sein, auch in einem Deckelfalz lösbar gehalten werden.

Nach der Erfindung ist als Membran ein aufblasbarer, mit seinem Mündungsrand gegenüber dem Rand der Öffnung für vom Behälterinneren verschiedenen Gasdruck im Bodenteil gasdicht festgelegter Ballon zu verwenden. Dieser ist zweckmäßig am Behälterboden fixiert.

Mit dem angesaugten Blut gelangen häufig auch Schaum- und Fetteile in den Deckelraum, weshalb das Sieb bevorzugt mit Abstand vor der Auslauföffnung im Behälter gehalten ist. Dadurch wird einerseits erreicht, daß die Membran bei Druckluftanwendung nur bis zur Anlage an das Sieb gedrückt werden und in der Auslaßöffnung daher nicht verletzt werden kann. Andererseits wird nach dem Umdrehen des Behälters, wenn also die Auslauföffnung nach unten weist, zwischen Sieb und Auslaufstutzen eine Restmenge Blut zurückgehalten, auf der die Fetteile und der Schaum schwimmen, die dann leicht verworfen werden können.

Durch einen entsprechend geformten Saugstutzen als Einlauföffnung wird die Körperflüssigkeit an der Innenwand des Deckelteils und der Membran so eingeleitet, daß sie automatisch herunterfließt, was gemäß einer Weiterbildung der Erfindung durch eine tangential zur Innenwand des Deckelteils mündende Einlauföffnung erreicht werden kann. Dieses glatte Einlaufen des Blutes wird unterstützt, wenn die Einlauföffnung aus einem trompetenartig erweiterten oder T-förmigen Mundstück besteht, so daß die Strömung des in das Behälterinnere gelangenden Blutes verlangsamt ist.

Das erfindungsgemäße Autotransfusionsgerät schont das zu transfundierende Blut sowie den Patienten auf bestmögliche Weise: Während des Blutansaugens entweicht, nämlich die mitangesaugte Luft durch den an der Auslauföffnung angeordneten Unterdruckanschluß und wird nicht traumatisierend durch das gesammelte Blut hindurchgesaugt; diese Verwendungsart hat gegenüber einem Unterdruckanschluß im Bodenraum - bei dem die Bluttraumatisierung ebenfalls vermieden wird - den Vorteil, daß größere Mengen mitangesaugter Luft die Blutaufnahmekapazität des Autotransfusionsgerätes nicht vermindern. Nach beendeter Aspiration eventuell noch vorhandene Luft im Deckelraum kann vor der Retransfusion durch Einbringung von Druckfluid in den Bodenraum zunächst ausgetrieben werden, wobei Blutgerinsel und grobe Gewebeteile von dem Sieb zurückgehalten werden.

Der Behälter und die Membran können unabhängig voneinander aus verschieden elastischen, blutfreundlichen Werkstoffen, wie Polyurethan, Polyvinylchlorid, Silicon-Gummi, Polyäthylen u.ä., hergestellt werden.

Natürlich ist es möglich, gerinnungshemmende Flüssigkeiten in den Behälter einzubringen, ohne diese durch den Blutsauger aufsaugen zu müssen. Hierzu bietet sich ein der Einlaßöffnung vorgeschalteter Zwei- oder Dreiwegehahn oder eine weitere Einlaßöffnung im Deckelteil an. Der Dreiwegehahn, bzw. ein T- bzw. Y-Stück vor der patientenseitigen Einlauföffnung ermöglicht zudem ein gleichzeitiges Anschließen von zwei Autotransfusionsgeräten an die Blutansaugleitung, so daß entweder mit erhöhter Saugleistung bzw. ununterbrochen Blut angesaugt werden kann, indem mindestens ein Autotransfusionsgerät ständig an die Ansaugleitung angeschlossen und das andere Gerät nach Kapazitätsausschöpfung abgeklemmt und durch ein frisches Gerät ausgetauscht werden kann.

Mit dem erfindungsgemäßen Autotransfusionsgerät ist es also möglich, mehrere wichtige Kriterien gemeinsam zu erfüllen, nämlich schonende Aspiration, aktive Retransfusion, Zurückhaltung aller groben Schadstoffe, Zurückhaltung von Fett und Schaum sowie mögliche Wiederverwendbarkeit, verbunden mit einer sicheren und ständigen Funktionsbereitschaft.

Es versteht sich, daß die Merkmale der Unteransprüche in beliebig sinnvoller Kombination mit den Merkmalen der übergeordneten Ansprüche ebenfalls den Gegenstand der Erfindung bilden.

Weitere Einzelheiten, Merkmale und Vorteile des Gegenstandes der Erfindung ergeben sich aus der nachfolgenden Beschreibung der zugehörigen Zeichnung, in der eine bevorzugte Ausführungsform eines erfindungsgemäßen Autotransfusionsgerätes dargestellt worden ist. In der Zeichnung zeigen:
- Fig. 1: ein Autotransfusionsgerät in Seitenansicht als Prinzipdarstellung gemäß der Stammanmeldung EP-A-0 116 352 sowie
- Fig. 2: ein erfindungsgemäße Autotransfusionsgerät im Längsschnitt in Teilansicht.

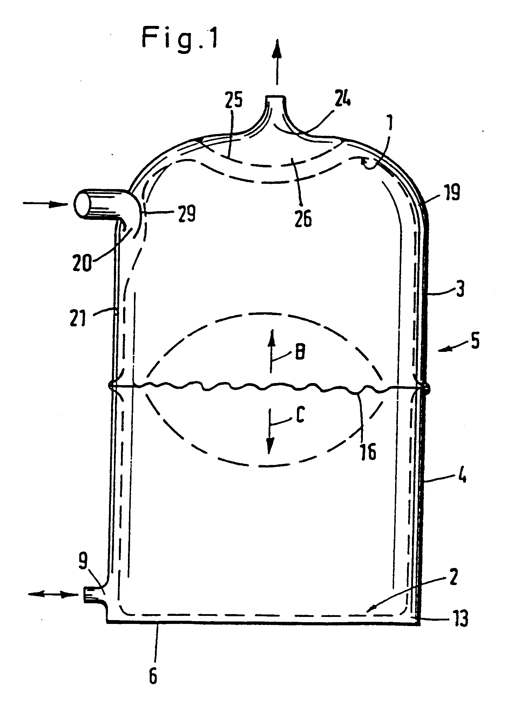

Fig. 1 zeigt den wesentlichen Teil eines Autotransfusionsgerätes in Form eines im ganzen mit 5 bezeichneten, aus sterilisierbarem Kunststoff oder Glas bestehenden Behälter, der als Einmal-Behälter ausgeführt sein kann. Der Behälter weist einen zylindrischen oder rechtwinkligen Querschnitt auf und besteht aus einem Bodenteil 4 und einem mit dessen oberem Rand fluiddicht verbundenen Deckelteil 3. Der Behälter 5 ist unterdruckstabil und über eine Auslauföffnung 24 im Deckelteil 3 oder eine Öffnung 9 im Bodenteil 4 evakuierbar. Im Deckelteil 3 ist in ausreichendem Abstand von der Auslauföffnung 24 eine Einlauföffnung 20 im Bereich eines trompetenförmigen Mundstücks 29 tangential zur Innenwand 21 des Deckelteils 3 angeordnet, so daß vom Patienten angesaugte Körperflüssigkeit an dieser Innenwand durch Gravitation ohne Schaumbildung hinabläuft. Ein Sieb 25 zum Zurückhalten von im Blut mitgeführten Grobstoffen deckt die Auslauföffnung 24 mit Abstand vom Behälterinnenraum her ab. Die Öffnung 9 ist gasdicht verschließbar und ermöglicht nach Entfernen des Verschlusses das Eindringen atmophärischer Luft oder von Druckluft in das Behälterinnere. Eine z.B. durch Druck verformbare, für die Körperflüssigkeit undurchlässige Membran 16 ist zwischen dem Bodenteil 4 und dem Deckelteil 3 randseitig gasdicht derart festgelegt, daß sie den Behälter 5 in einen gasgefüllten Bodenraum 13 und einen demgegenüber abgedichteten Deckelraum 19 - zum Aufnehmen der Körperflüssigkeit - unterteilt. Unter dem Einfluß eines Gasdruckes kann sich die Membran 16, wie gestrichelt dargestellt, an die Innenkontur 1 des Deckelteils 3 sowie alternierend an die Innenkontur 2 des Bodenteils 4 im wesentlichen anlegen. Selbstverständlich sind alle Zwischenpositionen der Membran 16 zwischen diesen beiden Extrempositionen einnehmbar, was durch weitere gestrichelte Linien und die Richtungspfeile B und C dargestellt ist.

Die Verbindung zwischen dem Membranrand und dem Boden- und Deckelteil kann durch Kunststoffverschweißen unter Bildung eines einteiligen Einmal-Behälters erfolgen.

Bei völlig mit Blut gefüllten Behältern nimmt die Membran 16 im wesentlichen die Form der Innenkontur 2 ein. Dann wird die Zulaufleitung vom Patienten dicht verschlossen und ggf. patientenseitig abgeklemmt. Nach dem Entfernen einer nicht dargestellten Vakuumleitung, die entweder an die Auslaßöffnung 24 oder an die Öffnung 9 angeschlossen ist, kann durch Einlassen von Druckluft durch die Öffnung 9 und Anschließen eines Retransfusionsbesteckes an die Öffnung 24 die im Deckelraum 3 und in den nachgeordneten Systembereichen noch vorhandene Luft ausgetrieben werden. Dabei hält das Sieb 25 Grobbestandteile zurück. Nach dem Auf-den-Kopf-Drehen des Behälters 5 haben sich nach einer Beruhigungszeit alle Leichtstoffe des Blutes, wie noch nicht ausgetriebene Luftbläschen und Fettzellen, unter der - dann oben liegenden - Membran 16 gesammelt. Beim anschließenden Einlassen von Umgebungs- oder Druckluft durch die Öffnung 9 strömt das Blut so lange zum Patienten zurück, bis die Membran 16 sich an die Kontur 1 des Deckelteils 3 sowie an das Sieb 25 ganz angelegt hat. Dabei verbleiben zwischen Membran 16 und Sieb 25 im Deckelraum 19 nur noch vom Sieb zurückgehaltene Grobbestandteile, während Feinbestandteile, wie die erwähnten Luftblasen oder Fettzellen dann konzentriert im Raum 26 zwischen der Auslauföffnung 24 und dem Sieb 25 angesammelt sind und nicht mehr zum Patienten gelangen können.

Ein wiederverwendbares Autotransfusionsgerät kann einen verbreiterten Fuß 6 sowie einen radial abstehenden Schraubrand am offenen Ende des Bodenteils und einen damit korrespondierenden umlaufenden Rand am offenen Ende des Deckelteils aufweisen.

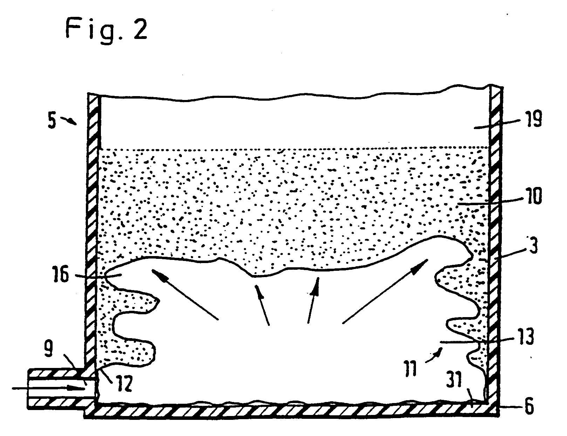

Fig. 2 zeigt eine erfindungsgemäße Ausführung der Erfindung, bei der die Membran 16 die Form eines aufblasbaren Ballon 11 hat. Der Ballon 11 ist am Boden 31 des Behälters 5 in geeigneter Weise, beispielsweise durch Verkleben, fixiert. Der Mündungsrand 12 des aufblasbaren Ballons 11 ist in der Nähe der bodennahen Öffnung 9 umlaufend und gasdicht befestigt. Hierzu kann der Mündungsrand 12 um eine Anschlußtülle der Öffnung 9 umgelegt und mit einer Steckkappe oder dgl. gehalten werden. Damit reduziert sich das Bodenteil auf diese Steckkappe.

Die Gefahr einer Traumatisierung des Blutes 10 beim Einleiten in den Behälter 5 wird (wie bereits im Zusammenhang mit Fig. 1 erklärt) durch eine entsprechende Gestaltung der Einlauföffnung 20 mit Mundstück 29 sichergestellt.

## Patentansprüche

1. Autotransfusionsgerät für Blut oder dergleichen Körperflüssigkeit, bestehend aus
einem evakuierbaren, unterdruckstabilen, ein Bodenteil (4) und ein Deckelteil (3) aufweisenden Behälter (5) mit, einer im Deckelteil angeordneten Einlauföffnung (20) für die Körperflüssigkeit,
einer Auslauföffnung (24) im Deckelteil (3) für die Körperflüssigkeit mit einem die Auslauföffnung abdeckenden Sieb (25) sowie,
einer Öffnung (9) im Bodenteil (4) zum Herstellen einer Gasströmungsverbindung zu einem mit Gas befüllbaren Bodenraum 13, in welchem Behälter (5)
eine durch Druck verformbare, für die Körperflüssigkeit undurchlässige Membran (6) vorgesehen ist, die
den Behälter (5) in den gasgefüllten Bodenraum (13) und einen demgegenüber abgedichteten Deckelraum (19) zum Aufnehmen der Körperflüssigkeit unterteilt und
unter dem Einfluß des Gasdrucks bzw. der Körperflüssigkeit sowohl an die Innenkontur (1) des Deckelteils (3) als auch an die Innenkontur (2) des Bodenteils (4) im wesentlichen anlegbar ist,
**dadurch gekennzeichnet,** daß
die Membran (16) ein aufblasbarer, mit seinem Mündungsrand (12) gegenüber dem Rand der Öffnung (9) des Behälters (5) gasdicht festgelegter Ballon ist.

2. Gerät nach Anspruch 1, dadurch gekennzeichnet, daß der Behälter (5) mit der Membran (16) als einteiliger Einmal-Behälter, insbesondere aus Kunststoff, ausgebildet ist.

3. Gerät nach Anspruch 1 oder 2, dadurch gekennzeichnet, daß die Einlauföffnung (20) tangential zu der Innenwand (21) des Deckelteils (3) mündet.

4. Gerät nach einem der Ansprüche 1 bis 3, dadurch gekennzeichnet, daß die Einlauföffnung (20) aus einem T-förmigen Mundstück besteht.

5. Gerät nach einem der Ansprüche 1 bis 4, dadurch gekennzeichnet, daß die Einlauföffnung (20) aus einem trompetenartig erweiterten Mundstück besteht.

6. Gerät nach einem der Ansprüche 1 bis 5, dadurch gekennzeichnet, daß der Ballon (11) am Boden (31) des Behälters (5) fixiert ist.

7. Gerät nach einem der Ansprüche 1 bis 6, dadurch gekennzeichnet, daß der Mündungsrand (12) um eine Anschlußfülle der Öffnung (9) umgelegt u. mit einer Steckkappe o.dgl. gehalten wird.

## Claims

1. Autotransfusion device for blood or similar body fluid, comprising a container (5) which can be evacuated, is stable at low pressure and has a bottom part (4) and a top part (3), this container (5) having an inflow opening (20) arranged in the top part for the body fluid, an outflow opening (24) in the top part (3) for the body fluid with a filter (25) covering the outflow opening, and having an opening (9) in the bottom part (4) for providing a gas flow connection to a bottom space (13) which can be filled with gas, in which container (5) there is provided a diaphragm (6) which is deformable by pressure, is impermeable to the body fluid, subdivides the container (5) into the gas-filled bottom space (13) and a top space (19), which is sealed off with respect thereto, for receiving the body fluid and can substantially be applied both against the inner contour (1) of the top part (3) and against the inner contour (2) of the bottom part (4) under the influence of the gas pressure or of the body fluid, characterized in that the diaphragm (16) is an inflatable balloon which is fixed in gas-tight manner by means of its mouth edge (12) with respect to the edge of the opening (9) of the container (5).

2. Device according to Claim 1, characterized in that the container (5), together with the diaphragm (16), is constructed as a one-part disposable container, in particular of plastics material.

3. Device according to Claim 1 or 2, characterized in that the inflow opening (20) opens tangentially with respect to the inner wall (21) of the top part (3).

4. Device according to one of Claims 1 to 3, characterized in that the inflow opening (20) comprises a T-shaped mouthpiece.

5. Device according to one of Claims 1 to 4, characterized in that the inflow opening (20) comprises a mouthpiece which is widened in the manner of a trumpet.

6. Device according to one of Claims 1 to 5, characterized in that the balloon (11) is fixed to the bottom (31) of the container (5).

7. Device according to one of Claims 1 to 6 characterized in that the mouth edge (12) is laid around a connection spout of the opening (9) and is held by means of a push-on cap or the like.

## Revendications

1. Appareil d'autotransfusion pour sang ou liquide corporel similaire, se composant
d'un récipient (5) pouvant être mis sous vide, stable à la dépression et présentant une partie de fond (4) et une partie de couvercle (3) avec un orifice d'entrée (20) ménagé dans la partie de couvercle pour le liquide corporel,
d'un orifice de sortie (24) dans la partie de couvercle (3) pour le liquide corporel avec un filtre (25) recouvrant l'orifice de sortie, ainsi
que d'un orifice (9) dans la partie de fond (4) pour générer une liaison d'écoulement du gaz à une chambre de fond (13) remplie de gaz, récipient (5) dans lequel est prévue une membrane (6) imperméable au liquide corporel, déformable sous la pression, qui divise le récipient (5) en chambre de fond (13) et en chambre de couvercle (19) étanche par rapport à celle-ci pour recueillir le liquide corporel et qui peut s'appliquer essentiellement, sous l'influence de la pression du gaz resp. du liquide corporel, aussi bien contre le contour interne (1) de la partie de couvercle (3) que contre le contour interne (2) de la partie de fond (4),
caractérisé en ce que
la membrane (16) est un ballon gonflable fixé de manière à ce que son bord d'embouchure (12) soit étanche au gaz par rapport au bord de l'ouverture (9) du récipient (5).

2. Appareil selon la revendication 1, caractérisé en ce que le récipient (5) est réalisé en une partie, avec la membrane (16) comme un récipient en matière plastique, jetable.

3. Appareil selon la revendication 1 ou 2, caractérisé en ce que l'orifice d'entrée (20) débouche de manière tangentielle par rapport à la paroi interne (21) de la partie de couvercle (3).

4. Appareil selon l'une des revendications 1 à 3, caractérisé en ce que l'orifice d'entrée (20) se compose d'une embouchure en forme de T.

5. Appareil selon l'une des revendications 1 à 4, caractérisé en ce que l'orifice d'entrée (20) se compose d'une embouchure s'élargissant en forme de trompette.

6. Appareil selon l'une des revendications 1 à 5, caractérisé en ce que le ballon (11) est fixé au fond (31) du récipient (5).

7. Appareil selon l'une des revendications 1 à 6, caractérisé en ce que le bord (12) de l'embouchure est placé autour d'une douille de raccordement de l'ouverture (9) et est maintenu par un capuchon enfichable ou similaire.
